Europäisches Patentamt

European Patent Office

Office européen des brevets

(19)

(11) Publication number : **0 652 008 A1**

(12) **EUROPEAN PATENT APPLICATION**

(21) Application number : 94305331.4

(22) Date of filing : 20.07.94

(51) Int. Cl.⁶ : **A61K 31/47, A61K 9/127**

(30) Priority : 23.07.93 CA 2101241

(43) Date of publication of application :
10.05.95 Bulletin 95/19

(84) Designated Contracting States :
AT BE CH DE DK ES FR GB IT LI NL SE

(71) Applicant : **THE MINISTER OF NATIONAL DEFENCE OF HER MAJESTY'S CANADIAN GOVERNMENT**
**National Defence Headquarters**
**101 Colonel By Drive**
**Ottawa Ontario, K1A 0K2 (CA)**

(72) Inventor : **Wong, Jonathan P.**
**43 Robinson Cresent, S.E.**
**Medicine Hat, Alberta T1B 3G3 (CA)**
Inventor : **Di Ninno, Vincent L.**
**P.O. Box 1328**
**Redcliff, Alberta T0J 2P0 (CA)**
Inventor : **Cherwonogrodzky, John W.**
**107 Sprague Way S.E.**
**Medicine Hat, Alberta T1B 3L5 (CA)**

(74) Representative : **Perry, Robert Edward**
**GILL JENNINGS & EVERY**
**Broadgate House**
**7 Eldon Street**
**London EC2M 7LH (GB)**

(54) Liposome-encapsulated ciprofloxacin.

(57)    Despite its potency and broad spectrum antibacterial activity, the therapeutic effectiveness of ciprofloxacin is limited in that it has a short elimination half-life and its potency against some bacteria may be compromised by the ability of some intracellular bacteria to reside and multiply within the human host cells where they are protected from the antibiotic. These problems can be solved by encapsulating the antibiotic within the microscopic lipid vesicles called liposomes, which protect the drug from dilution and elimination from the body. Moreover, the liposome-encapsulated antibiotic is naturally taken up by phagocytic cells which results in the delivery of the drug to intracellular sites of infection.

EP 0 652 008 A1

This invention relates to a method of improving the effectiveness of an antibiotic, and specifically to a method of improving the therapeutic effectiveness of ciprofloxacin.

The invention also relates to a pharmaceutically active ciprofloxacin composition.

New antibiotics which are more powerful and which display a broad spectrum activity against a number of different bacteria than existing compounds are continuously being developed. Among these new generations of antibiotics are a family of antibiotics called quinolones.

Quinolones are very powerful antibiotics because they are structurally unrelated to the conventional antibiotics such as penicillins, cephalosporins and tetracyclines. Therefore, many of the pathogenic bacteria which are resistant to the conventional antibiotics are highly susceptible to the bactericidal actions of quinolones. Moreover, the quinolones have significantly higher bioavailability rates than conventional penicillins, ranging from 75% to approaching 100% (compared to 45-50% for penicillin G).

Ciprofloxacin is the most potent member of quinolones. As with other quinolones, ciprofloxacin's bactericidal action is achieved through inhibition of bacterial DNA gyrase system, which is an essential component of the bacterial DNA replication system. The antibacterial activity of ciprofloxacin is further enhanced by its unique cyclopropyl side chain. In addition to its potency, ciprofloxacin has broad spectrum activity and is extremely effective against most gram negative and some gram positive pathogenic bacteria, with low occurrence of resistance to the drug.

Despite its potency and broad spectrum activity, the therapeutic effectiveness of ciprofloxacin as an antibiotic is limited by the following factors:

(a) ciprofloxacin has a short elimination half-life of only 4.0 hours (compared to 10.0 hours for pefloxacin, another quinolone). Once administered, the drug is rapidly diluted and cleared from the body, thus making it difficult to reach therapeutic concentrations;

(b) ciprofloxacin has been known to increase serum levels and hence the toxicity of concurrently administered theophylline. One study found that 20 of 33 patients taking both drugs developed increases of theophylline, many to the toxic range;

(c) the potency of ciprofloxacin against some pathogenic bacteria may be compromised by the ability of some intracellular bacteria to reside and multiply within human host cells, and hence remain protected from the powerful antibiotic.

The object of the present invention is to solve the above identified problems by providing a method of improving the therapeutic effectiveness of ciprofloxacin.

According to one aspect, the invention relates to an antibiotic formulation comprising ciprofloxacin encapsulated within liposomes.

According to a second aspect, the invention relates to a method of preparing liposome-encapsulated ciprofloxacin comprising the step of freeze drying a mixture of liposomes and ciprofloxacin.

## BACTERIAL STRAINS

*Francisella tularensis* is an extremely pathogenic Gram-negative bacterium which can be transmitted from wildlife to humans by a tick vector. It has been projected that some strains can cause a 5-30% mortality in humans with as little as 10-50 organisms and, for those surviving infection, recovery from this debilitating disease may take 3-6 months.

The ineffectiveness of antibiotics to eliminate *Francisella tularensis* may lie in the bacterium's ability to be an intracellular parasite, residing within reticuloendothelial cells. Some antibacterial agents are limited in their *in vivo* action against pathogens because of their poor tissue penetrability. Liposomes are microscopic lipid vesicles that are effective for the targeting of antimicrobial agents to the intracellular sites of infection. Because of this attribute, liposomes are an excellent drug delivery system for the prevention and treatment of infectious diseases caused by intracellular parasites.

*Francisella tularensis* LVS (available from ATCC) was grown on synthetic agar medium with a pH of 6.5 at 37° with 5% $CO_2$. Growth on this medium increased the virulence of the bacteria in mice. Purity of the cultures was confirmed by Gram-staining and by agglutination with commercial antiserum.

## LIPOSOMES

Liposomes for the encapsulation of ciprofloxacin were prepared using a modification of the freeze drying method described by Kirby and Gregoriadis Vol.1, Liposome (see Technology, pp 17-27, CRC Press, 1984). Negatively-charged multilamellar liposomes were prepared from phosphatidylcholine:phosphatidylserine:cholesterol in a molar ratio of 7:1:2, in the following manner. A screw-capped tube containing a total of 20 µmoles of the lipids mixed with 1 ml of chloroform:methanol (2:1) was heated in a heating block with the temperature set at 45° until the lipids formed a thin lipid film at the bottom of the tube. In order to facilitate the evaporation of the organic solvent as well as to prevent oxidation of the lipids, the contents of the tube were continuously purged throughout the procedure with a gentle stream of dry nitrogen. The resulting lipid film was further dried for 30 minutes in a vacuum oven to remove any residual organic solvent.

The dried lipids were subsequently rehydrated with 2 ml of 0.22 μm membrane-filtered distilled water containing 40 mg of ciprofloxacin obtained from Miles Canada Inc., Etobicoke, Ont. The contents of the tube were mixed vigorously by vortexing, followed by heating at 45°C. The vortexing-heating cycles were repeated under nitrogen until all the dried lipids were completely dislodged from the sides of the tube. The lipid-ciprofloxacin mixture was then sonicated in a bath type sonicator for approximately 4 minutes, and was rapidly frozen in a dry ice-methanol mixture. The sample was then freeze-dried overnight in a lyophilizer (Virtis Company Inc., Gardiner, N.Y.). The dried material was then reconstituted with 500 μl of distilled water. The reconstituted liposomes were then vortexed for 2 - 3 minutes and were left undisturbed at room temperature for 1 hour. The liposomes were washed with 8 ml of distilled water, then ultracentrifuged at 125,000 x g for 30 minutes. The pellet was rewashed with 8 ml of distilled water and the pellet from the final ultracentrifugation step was reconstituted to the appropriate concentration to be immediately used for administration into mice.

TESTING

All administrations of bacteria, liposomes and antibiotic into mice were by the intravenous or intranasal routes. Intravenous injections were given through the tail vein (0.2 ml per mouse) and intranasal inoculations were administered to anaesthetized mice through the nostrils using a micropipettor (50 μl per mouse). For all bacterial challenges, BALB/c mice were infected with 10 $LD_{50}$ of *Francisella tularensis*, corresponding to 10 colony forming units (CFU) of the bacteria given by the intranasal route or 100 CFU by the intravenous route. The CFU administered to these mice were verified retrospectively by plating dilutions on synthetic agar medium.

For the prophylactic study, groups of mice were pretreated with a single dose of ciprofloxacin (1 mg per mouse), liposomal ciprofloxacin (1 μmole lipid equivalent of liposomes containing 1 mg ciprofloxacin) or sham liposomes (1 μmole lipid), by the intravenous or intranasal route. At day 1, 2, 3 and 7 post administration, these mice were challenged with 10 $LD_{50}$ of the bacteria using the same routes of administration. At day 14 post infection, the number of mice that had survived the infection was then recorded.

For the treatment, groups of mice were infected with 10 $LD_{50}$ of the bacteria. At day 1, 2, 3, 4, or 7 post infection, the mice were treated with ciprofloxacin, liposomal ciprofloxacin (LIP-CIP) or sham liposomes as described before using the same routes of administration as infection. At day 14 post infection, the number of survivors were then recorded.

RESULTS

Mice, when given single doses of either free unencapsulated ciprofloxacin or sham liposomes before infection, succumbed to 10 $LD_{50}$ of tularemia in a manner similar to the control untreated mice (see Table 1).

## TABLE 1

Protection of BALB/c mice from infection with 10 $LD_{50}$ of
*Francisella tularensis*

% SURVIVALS AT DAY 14 POST INFECTION

| GROUP | TIME BEFORE* INFECTION (DAYS) | ROUTE OF ADMINISTRATION ** | |
| --- | --- | --- | --- |
| | | INTRAVENOUS | INTRANSAL |
| Saline Control | 1 | 8 | 16 |
| | 2 | 0 | 0 |
| | 3 | 0 | 12 |
| | 7 | 0 | 0 |
| Sham liposomes | 1 | 0 | 0 |
| | 2 | 0 | 0 |
| | 3 | 0 | 0 |
| | 7 | 0 | 0 |
| Ciprofloxacin | 1 | 8 | 0 |
| | 2 | 0 | 0 |
| | 3 | 12 | 0 |
| | 7 | 0 | 12 |
| Liposomal Cipro | 1 | 92 | 92 |
| | 2 | 100 | 83 |
| | 3 | 25 | 100 |
| | 7 | 0 | 63 |

\*       Groups of mice were pretreated with saline, sham
liposomes (without antibiotic), ciprofloxacin or liposomal
ciprofloxacin, and at time interval noted, infected with 10
$LD_{50}$ of *Francisella tularensis*.

\*\*       Mice were pretreated and infected by the same
route of administration, either intravenously or
intranasally.

In comparison, mice which were given a single dose of liposomal ciprofloxacin at day 1 or 2 before infection were highly protected from the bacterial challenge, with survival rates ranging from 83% to 100% (P < 0.004). Even when the mice were given a single intranasal dose of liposomal ciprofloxacin at day 3 before infection, the protection rate was significant at 63% (P < 0.04). To determine the efficacy of liposomal ciprofloxacin to eradicate infectious pathogens which may hide within phagocytic cells of organs that constitute the reticuloendothelial system, the liver, lung and spleen from the infected mice which had been pretreated with a single dose of liposomal ciprofloxacin were removed, homogenized and assayed for viable *Francisella tularensis*. No detectable viable *Francisella tularensis* (< 200 CFU) was recovered from the organs assayed (see Table 2).

## TABLE 2

Organ recovery of viable *Francisella tularensis* from BALB/c mice following pretreatment with a single dose of liposomal ciprofloxacin

| GROUP | ORGAN | *Francisella tularensis* recovered (Colony forming units, CFU) |
|---|---|---|
| Untreated control | Spleen | $4.7 + 1.3 \times 10^6$ |
| | Liver | $2.6 + 0.2 \times 10^7$ |
| | Lung | $4.0 + 0.6 \times 10^7$ |
| Liposomal Ciprofloxacin* | Spleen | $< 2 \times 10^2$ |
| | Liver | $< 2 \times 10^2$ |
| | Lung | $< 2 \times 10^2$ |

* Mice were pretreated with a single dose of liposomal ciprofloxacin by the intranasal route, and 24 hrs later, they were intranasally infected with 10 $LD_{50}$ of *Francisella tularensis*. The mice were then sacrificed at day 7 post infection, and the various organs were isolated, homogenized and assayed for viable *Francisella tularensis* by plating on synthetic agar medium.

In contrast, a corresponding group of control mice which did not receive pretreatment with liposomal ciprofloxacin showed very high number of viable *Francisella tularensis* organisms ($> 10^6$ CFU) in all of the organs assayed. These results indicate the potency of liposomal ciprofloxacin in protecting mice against lethal infection with *Francisella tularensis*, and in the intracellular killing of the bacteria in organs which ordinarily harbour these microorganisms.

To compare the efficacy of free and liposomal ciprofloxacin in the treatment of mice already infected with 10 $LD_{50}$ of the bacteria, the infected mice were treated at day 1, 2, 3 and 7 post infection with a single dose of free, liposomal ciprofloxacin or sham liposomes. Free unencapsulated ciprofloxacin was totally ineffective for the treatment of infected mice (see Table 3).

TABLE 3

Treatment of mice infected with 10 $LD_{50}$ of *Francisella tularensis*

|  |  | % SURVIVALS AT DAY 14 POST INFECTION* | |
| --- | --- | --- | --- |
| GROUP | TIME AFTER INFECTION (DAYS) | ROUTE OF INFECTION AND TREATMENT | |
|  |  | INTRAVENOUS | INTRANSAL |
| Saline control | 1 | 0 | 12 |
|  | 2 | 0 | 0 |
|  | 3 | 0 | 0 |
|  | 7 | 0 | 12 |
| Ciprofloxacin | 1 | 0 | 50 |
|  | 2 | 25 | 0 |
|  | 3 | 0 | 25 |
|  | 7 | 12 | 0 |
| Liposomal Cipro | 1 | 75 | 83 |
|  | 2 | 88 | 100 |
|  | 3 | 0 | 63 |
|  | 7 | 0 | 50 |

\* Mice were infected with 10 $LD_{50}$ of *Francisella tularensis* and at various time post infection, were treated with a single dose of either ciprofloxacin of liposomal ciprofloxacin. The percentage survival rates were determined at day 14 post infection.

In contrast, liposomal ciprofloxacin was found to be highly effective in the treatment of the infected mice, particularly when administered within two days after the mice were infected. The survival rates in these groups of mice ranges from 77% to 100%. It was also observed that the intranasal route of administration for the treatment of these mice was found to be more effective than the intravenous route. The survival rates for the infected mice which were treated using this route of administration at day 4 and 7 post infection were 65 and 50%, respectively, compared to 0% by intravenous route of administration.

This study shows that free unencapsulated ciprofloxacin was ineffective in the prevention and treatment of mice against tularemia. In contrast, liposomal ciprofloxacin provided effective prophylaxis and treatment against infection caused by large lethal doses of the bacteria. These results suggest that the prophylactic and therapeutic effectiveness of this antibiotic can be dramatically enhanced by liposome-encapsulation, particularly when used against infectious diseases that are caused by bacteria that infect the phagocytic cells of the reticuloendothelial system.

**Claims**

1. An antibiotic formulation comprising ciprofloxacin encapsulated within liposomes.

2. An antibiotic according to claim 1, wherein said liposomes are multilamellar liposomes prepared from the lipids: phosphatidylcholine, phosphatidylserine and cholesterol.

3. An antibiotic according to claim 1, wherein the phosphatidylcholine, phosphatidylserine and cholesterol are present in the liposomes in a molar ratio of 7:1:2.

4. A method of preparing liposome-encapsulated ciprofloxacin comprising the step of freeze drying a mixture of liposomes and ciprofloxacin.

5. A method according to claim 4, including the steps of thoroughly mixing a suspension of lipids with ciprofloxacin, drying the resulting mixture, sonicating the dried mixture, freeze drying the sonicated mixture, reconstituting the dried mixture, and separating the liposome pellets thus produced.

6. A method according to claim 5, wherein the liposomes are multilamellar liposomes prepared from phosphatidylcholine, phosphatidylserine and cholesterol.

7. A method according to claim 6, wherein the phosphatidylcholine, phosphatidylserine and cholesterol are present in the liposomes in a molar ratio of 7:1:2.

8. A method according to claim 5, 6 or 7, wherein the suspension of lipids is produced by dissolving the lipids in a suitable solvent, drying the solution thus produced, and adding ciprofloxacin solution to the dried lipids.

EP 0 652 008 A1

**European Patent Office**

# EUROPEAN SEARCH REPORT

Application Number

EP 94 30 5331

## DOCUMENTS CONSIDERED TO BE RELEVANT

| Category | Citation of document with indication, where appropriate, of relevant passages | Relevant to claim | CLASSIFICATION OF THE APPLICATION (Int.Cl.6) |
|---|---|---|---|
| X | WO-A-91 09616 (YALE UNIVERSITY) | 1,2,4-6, 8 | A61K31/47 A61K9/127 |
| Y | * page 3, line 7 - page 4, line 25 * <br> * page 6, line 12 - page 7, line 31; claims 1-6,13 * <br> --- | 3,7 | |
| Y | EP-A-0 068 314 (CIBA-GEIGY AG) <br> * page 11, line 20 * <br> --- | 3,7 | |
| Y | PHARMAZIE IN UNSERER ZEIT, <br> vol.20, no.6, 1991 <br> pages 255 - 270 <br> W. RUBAS ET AL. 'Liposomen: Fortschritte in Herstellungs-Technologie und Therapie' <br> * table 4 * <br> ----- | 3,7 | |
| | | | TECHNICAL FIELDS SEARCHED (Int.Cl.6) |
| | | | A61K |

The present search report has been drawn up for all claims

| Place of search | Date of completion of the search | Examiner |
|---|---|---|
| MUNICH | 22 December 1994 | Foerster, W |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

8